# EUROPEAN PATENT SPECIFICATION

(11) **EP 3 020 805 B1**
(45) Date of publication and mention of the grant of the patent: **12.09.2018**
(21) Application number: 14822858.8
(22) Date of filing: 21.05.2014
(51) Int. Cl.: C12N 7/02, C07K 14/08, A61K 39/12

(54) **PRODUCTION METHOD FOR CULTURE CONTAINING VIRUS-LIKE PARTICLES**
HERSTELLUNGSVERFAHREN FÜR EINE KULTUR MIT VIRUSÄHNLICHEN PARTIKELN
PROCÉDÉ DE FABRICATION DE CULTURE CONTENANT DES PSEUDO-PARTICULES VIRALES

(30) Priority: 12.07.2013 JP 2013146242
(43) Date of publication of application: 18.05.2016
(73) Proprietor: Umn Pharma Inc., Akita-shi, Akita 010-1415 (JP); Vesikari, Timo, 33520 Tampere (FI); Blazevic, Vesna, 33520 Tampere (FI)
(72) Inventor: OKADA, Masahiro, Yokohama Kanagawa 222-0033 (JP); MUKAI, Akiko, Yokohama-shi Kanagawa 222-0033 (JP); NISHINO, Tomonori, Yokohama-shi Kanagawa 222-0033 (JP); ARINOBU, Daisuke, Yokohama-shi Kanagawa 222-0033 (JP); ITO, Hiroyuki, Yokohama-shi Kanagawa 222-0033 (JP); SATOH, Mamoru, Yokohama-shi Kanagawa 222-0033 (JP)
(74) Representative: J A Kemp
(86) International application number: PCT/JP2014/063424
(87) International publication number: WO 2015/004997

(56) References cited:
- WO-A1-98/21338
- WO-A1-2010/062757
- WO-A2-2008/113011
- JP-A- 2010 530 734
- JP-A- 2012 507 985

### SEQUENCE LISTING

<110> UMN Pharma Inc.
<120> method for producing culture containing virus-like particles
<130> FP-193PCT
<150> JP 2013/146242
   <151> 2013-7-12
<160> 1
<170> PatentIn Ver. 2.1
<210> 1
   <211> 539
   <212> PRT
   <213> Norwalk virus
<400> 1
   Met Lys Met Ala Ser Asn Asp Ala Asn Pro Ser Asp Gly Ser Thr Ala
   1               5                   10                  15
   Asn Leu Val Pro Glu Val Asn Asn Glu Val Met Ala Leu Glu Pro Val
               20                  25                  30
   Val Gly Ala Ala Ile Ala Ala Pro Val Ala Gly Gln Gln Asn Val Ile
           35                  40                  45
   Asp Pro Trp Ile Arg Asn Asn Phe Val Gln Ala Pro Gly Gly Glu Phe
       50                  55                  60
   Thr Val Ser Pro Arg Asn Ala Pro Gly Glu Ile Leu Trp Ser Ala Pro
   65                  70                  75                  80
   Leu Gly Pro Asp Leu Asn Pro Tyr Leu Ser His Leu Ala Arg Met Tyr
                   85                  90                  95
   Asn Gly Tyr Ala Gly Gly Phe Glu Val Gln Val Ile Leu Ala Gly Asn
               100                 105                 110
   Ala Phe Thr Ala Gly Lys Ile Ile Phe Ala Ala Val Pro Pro Asn Phe
           115                 120                 125
   Pro Thr Glu Gly Leu Ser Pro Ser Gln Val Thr Met Phe Pro His Ile
       130                 135                 140
   Ile Val Asp Val Arg Gln Leu Glu Pro Val Leu Ile Pro Leu Pro Asp
   145                 150                 155                 160
   Val Arg Asn Asn Phe Tyr His Tyr Asn Gln Ser Asn Asp Ser Thr Ile
                   165                 170                 175
   Lys Leu Ile Ala Met Leu Tyr Thr Pro Leu Arg Ala Asn Asn Ala Gly
               180                 185                 190
   Asp Asp Val Phe Thr Val Ser Cys Arg Val Leu Thr Arg Pro Ser Pro
           195                 200                 205
   Asp Phe Asp Phe Ile Phe Leu Val Pro Pro Thr Val Glu Ser Arg Thr
       210                 215                 220
   Lys Pro Phe Thr Val Pro Ile Leu Thr Val Glu Glu Met Ser Asn Ser
   225                 230                 235                 240
   Arg Phe Pro Ile Pro Leu Glu Lys Leu Tyr Thr Gly Pro Ser Ser Ala
                   245                 250                 255
   Phe Val Val Gln Pro Gln Asn Gly Arg Cys Thr Thr Asp Gly Val Leu
               260                 265                 270
   Leu Gly Thr Thr Gln Leu Ser Ala Val Asn Ile Cys Thr Phe Arg Gly
           275                 280                 285
   Asp Val Thr His Ile Ala Gly Ser His Asp Tyr Thr Met Asn Leu Ala
       290                 295                 300
   Ser Gln Asn Trp Asn Asn Tyr Asp Pro Thr Glu Glu Ile Pro Ala Pro
   305                 310                 315                 320
   Leu Gly Thr Pro Asp Phe Val Gly Lys Ile Gln Gly Leu Leu Thr Gln
                   325                 330                 335
   Thr Thr Arg Glu Asp Gly Ser Thr Arg Ala His Lys Ala Thr Val Ser
               340                 345                 350
   Thr Gly Ser Val His Phe Thr Pro Lys Leu Gly Ser Val Gln Tyr Thr
           355                 360                 365
   Thr Asp Thr Asn Asn Asp Phe Gln Thr Gly Gln Asn Thr Lys Phe Thr
       370                 375                 380
   Pro Val Gly Val Ile Gln Asp Gly Asn Asn His Gln Asn Glu Pro Gln
   385                 390                 395                 400
   Gln Trp Val Leu Pro Asn Tyr Ser Gly Arg Thr Gly His Asn Val His
                   405                 410                 415
   Leu Ala Pro Ala Val Ala Pro Thr Phe Pro Gly Glu Gln Leu Leu Phe
               420                 425                 430
   Phe Arg Ser Thr Met Pro Gly Cys Ser Gly Tyr Pro Asn Met Asn Leu
           435                 440                 445
   Asp Cys Leu Leu Pro Gln Glu Trp Val Gln His Phe Tyr Gln Glu Ala
       450                 455                 460
   Ala Pro Ala Gln Ser Asp Val Ala Leu Leu Arg Phe Val Asn Pro Asp
   465                 470                 475                 480
   Thr Gly Arg Val Leu Phe Glu Cys Lys Leu His Lys Ser Gly Tyr Val
                   485                 490                 495
   Thr Val Ala His Thr Gly Pro His Asp Leu Val Ile Pro Pro Asn Gly
               500                 505                 510
   Tyr Phe Arg Phe Asp Ser Trp Val Asn Gln Phe Tyr Thr Leu Ala Pro
           515                 520                 525
   Met Gly Asn Gly Ala Gly Arg Arg Arg Ala Leu
       530                 535

## Description

### Technical Field

The present invention relates to a method for producing culture containing virus-like particles by using the baculovirus-insect cell expression system.

### Background Art

Norovirus is a virus that causes acute gastroenteritis such as vomiting and diarrhoea. Norovirus causes food poisoning from eating oysters and the like, and orally infects humans via feces or vomited material.

It is known that particles called as virus-like particles (VLPs) that resemble viral particles are formed when a baculovirus vector incorporated with the structural protein coding region of norovirus genome is expressed in insect cells. Though VLPs resemble viral particles in appearance, they do not contain virus genome and is not infectious.

Norovirus vaccine has been recently developed using VLPs as antigens, and patent applications concerning it have been filed (Japanese Translation of PCT International Application Publication Nos. JP-T-2010-505766 and JP-T-2011-530295). In order to produce such norovirus vaccine, it is important that VLPs can be efficiently produced by using the baculovirus-insect cell expression system.

Conventionally, when protein was produced by using the baculovirus-insect cell expression system, the cells were collected after about 3 days from the start of the cultivation, disrupted, and extracted to purify the protein from the cell extract. For example, Patent Literature 1 discloses a method for producing hemagglutinin of influenza virus. In this method, the cells were collected after 72 hours from the start of the cultivation, and the cell pellet was disrupted with homogenizer in presence of a surfactant (Example 5).

Cell contents are released to the culture solution by death of the cells when they are cultivated for a longer time, so the process for disrupting the cells can be omitted. However, protease released from the dead cells may decompose a target protein, so cultivation for such a long time has not been performed.

### Citation List

### Patent Literature

Patent Literature 1:WO96/37624

### Summary of Invention

### Technical Problem

When VLPs are produced by the above conventional method, the process for disrupting the cells must be performed to collect the VLPs. However, a surfactant that is used in the disrupting process may have bad effect on the structure of the VLPs. Further, the disrupting process itself is complicated.

The object of the present invention is to solve the problems of the conventional production method for VLPs and to provide a more efficient means for producing VLPs.

### Solution to Problem

As the result of intensive studies for solving the above-described problems, the present inventors have found that even in the presence of proteases released from the dead cells VLPs are virtually not affected by the proteases. Thus, the cells can be cultivated for a long time and VLPs can be collected without the disrupting process.

Further, the present inventors have found that the purity of VLPs in the culture supernatant is high because contaminant proteins are decomposed by the proteases released from dead cells when the cells are cultivated for a long time.

The present invention has been completed based on the above findings.

Specifically, the present invention provides the following (1) to (3).
(1) A production method for culture supernatant containing virus-like particles, comprising: transforming insect cells with baculovirus vector containing a viral nucleic acid sequence from norovirus, wherein the viral nucleic acid sequence encodes protein that can form a virus-like particle; cultivating the insect cells until the viability of the insect cells reaches 10% or less; and obtaining the culture supernatant.
(2) The production method according to (1), wherein the cultivation period of the insect cells is 5 days or more.
(3) The production method according to (1) or (2),
wherein the viral nucleic acid sequence is a nucleic acid sequence encoding a norovirus structural protein VP1.

### Advantageous Effects of Invention

In the production method of the present invention, VLPs can be obtained without the process for disrupting the cells, so effect of a surfactant that is used in the disrupting process can be excluded. Further, high-purity VLPs can be obtained because substances such as proteins derived from the host cell are decomposed during the process of cultivation.

### Brief Description of Drawing

[Figure 1] Figure 1 is a photograph showing the results of electrophoresis of samples that cultivated for different periods of time.
[Figure 2] Figure 2 is a graph showing the temporal change of viable and total number of cultivated cells. A horizontal axis shows days from the infection of baculovirus and a vertical axis shows number of cells.
[Figure 3] Figure 3 is a graph showing the temporal change of diameter of cultivated cells. A horizontal axis shows days from the infection of baculovirus and a vertical axis shows diameter of cells.
[Figure 4] Figure 4 is a graph showing the temporal change of viability of cultivated cells. A horizontal axis shows days from the infection of baculovirus and a vertical axis shows viability of cells.

### Description of Embodiments

Hereinafter, the present invention will be described in detail.

The production method for culture supernatant containing virus-like particles of the present invention comprises: transforming insect cells with baculovirus vector containing a viral nucleic acid sequence from norovirus, wherein the viral nucleic acid sequence encodes protein that can form a virus-like particle; cultivating the insect cells until the viability of the insect cells reaches 10% or less and obtaining the culture supernatant.

The type of a virus to be used is not particularly limited. For example, such viruses as non-enveloped viruses that have the shape of a regular icosahedron can be used. Examples of such viruses include circovirus, parvovirus, polyomavirus, papillomavirus, adenovirus, picornavirus, astrovirus, birnavirus, reovirus, hepevirus, nodavirus, anellovirus, calicivirus ,and the like. Calicivirus (viruses belonging to the family Caliciviridae) is preferably used among these. Norovirus is the most preferable.

Norovirus in the present invention means a virus that belongs to the family Caliciviridae and the genus Norovirus. Norwalk virus is the only species of the genus Norovirus at present. However, if a new virus belonging to this genus is found in the future, it is included in "Norovirus" in the present invention.

A virus-like particle in the present invention means a hollow particle that has only viral outer shell without viral genome inside the shell.

The cultivation may continue until the viability of the insect cells reaches 10% or less, but the cultivation may continue until the lower viability, for example, 9% or less, 7% or less, 5% or less, 3% or less, or 1% or less. The viability can be measured by using a method such as trypan blue staining method, AlamarBlue staining method, or MTT method. In addition, devices that automatically analyze whether a cell is alive or dead are commercially available, so the viability may be measured by using such devices.

Although specific cultivation period may be determined in accordance with the cell viability, the viability usually reaches 10% or less at about 5 days after the start of cultivation (the infection of baculovirus), so cultivation for 5 days or more is preferable. However, cultivation period may be 6 days or more, 7 days or more, or 8 days or more so that viability may become lower. The upper limit of the cultivation period is not particularly limited, but the cultivation period is preferably within 10 days.

In a conventional method, after cultivation, the cells collected from the culture were disrupted and extracted to obtain the target protein (VLPs) from the cell extract. However, in the present invention, VLPs can be obtained without the processes for disrupting and extracting the cells because the VLPs are released to the culture.

Except for the cultivation period and the unnecessity of the disrupting process described above, the production method of the present invention can be performed in the same manner as the known production method for a specific protein by using the baculovirus-insect cell expression system (for example, the method described in International Publication WO96 / 37624).

The nucleic acid sequence of the virus to be used is not particularly limited as long as it can form a VLP. For example, if the virus is norovirus, a nucleic acid sequence encoding VP1 can be used. Further, if the virus is norovirus, not only a nucleic acid sequence encoding VP1 but also a nucleic acid sequence encoding VP2 may be used.

The nucleic acid sequences of the virus to be used are published in such a database as GenBank. For example, registered numbers of the sequences of norovirus include M87661 of Norwalk virus, L07418 of Southampton virus, U04469 of Desert Shield virus, AB042808 of Chiba virus, U07611 of Hawaii virus, U70059 of Snow Mountain virus, AY032605 of Maryland virus, AB031013 of Seto virus, AF145896 of Camberwell, X86557 of Lordsdale virus, AJ004864 of Grimsby virus, U22498 of Mexico virus, AY502023 of Houston virus, and AY652979 of Parris Island.

The baculovirus vector which can be used includes pFastBac vector (Invitrogen) and BD BaculoGold (BD Biosciences).

The insect cell which can be used includes Sf9 cell and HighFive cell.

The cultivation of transformed insect cells can be performed according to the cultivation method generally used in the cultivation of insect cells. The medium which can be used include PSFM medium, SF900II medium, and SF900III medium. The cultivation temperature is preferably 20 to 30 °C, and more preferably 26 to 28 °C.

### EXAMPLES

The present invention will be explained more specifically with reference to the following Examples. However, the present invention is not limited thereto.

### Example 1: Cell cultivation

### (1) Experimental methods

### 1 Preparation of recombinant baculovirus

A cDNA encoding VP1, whose amino acid sequence is shown in SEQ ID NO.: 1, of a norovirus strain classified as GII-4, which was collected from an inpatient in University of Tampere, was incorporated into a transfer plasmid (pFastBac, Invitrogen). Then, the transfer plasmid incorporated with the cDNA was introduced into DH10Bac (Invitrogen), which is an Escherichia coli having a baculovirus genome, and the cDNA encoding the norovirus VP1 was incorporated into the baculovirus genome DNA by homologous recombination. The baculovirus genome DNA was extracted from the Escherichia coli, purified, and introduced into insect cells (expresSF+cells, Protein Sciences). The insect cells were cultivated, and from the culture supernatant, recombinant baculovirus was obtained.

### 2 Cultivation to express the GII-4 VP1

The recombinant baculovirus (MOI=1) was added to the insect cells (expresSF+cells) (1x10⁶ / mL). The cells (expresSF+cells) were cultivated in PSFM medium at 27 °C. After the infection of baculovirus, 1 ml of the culture solution was collected everyday and centrifuged at 5000 x g for 5 minutes to separate the culture supernatant from the cell pellet.

### 3 Electrophoresis

The culture supernatant was mixed with 250 µL of 5 x DB (300 mmol/L Tris-HCl pH6.8, 50% glycerol, 10% SDS, 0.5% Bromo Phenol Blue, 500 mmol/L DTT). The cell pellet was dissolved in 1250 µL of 1 x DB, which is prepared by diluting 5 x DB to 5 times with distilled water. After both were heated at 95 °C for 5 minutes, 10 µL of them were applied on each lane and electrophoresed at 200 V for 35 minutes.

The electrophoresed gel was soaked in fixing solution (25% methanol, 10% acetic acid, 10% trichloroacetic acid (TCA)) and shaken for 5 minutes. Then, it was stained by soaking in CBB stain solution (0.1% CBB, 7.7 mmol/L ethanol, 1.75 mmol/L acetic acid) and shaking for 1 hour, and destained with destaining solution (10% acetic acid) overnight. The gel image was obtained by using a scanner. SeeBlue prestained standard (Invitrogen) was used as a molecular weight marker.

### (2) Experimental results

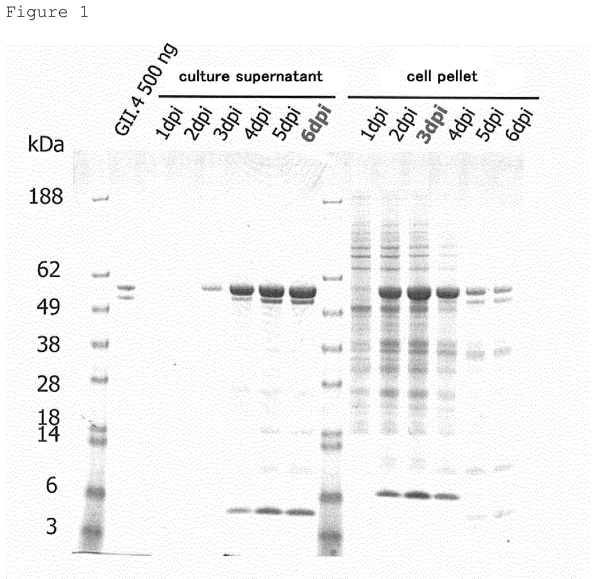

Results of electrophoresis are shown in Figure 1. In the figure, "dpi" is short for "days post infection" and represents how many days the sample was cultivated after the infection. The purified GII-4 VP1 was diluted with 1 x DB to 50 ng/µL and 10 µL of this was applied to the second left lane (GII-4 500ng).

As shown in the figure, while the amount of GII-4 VP1 in the culture supernatant, which was detected as a band of about 60k Da, increased with the cultivation days, the amount of GII-4 VP1 in the cell pellet decreased. This suggests that the VLPs released to the culture supernatant increased by the increase of the dead cells with the cultivation days. Thus, it is believed that an enough amount of VLPs can be obtained without the process for disrupting the cells if the cultivation continues for about five days.

Further, the culture supernatant cultivated for 6 days contains a great amount of the GII-4 VP1, so it is believed that the proteases released from dead cells hardly act on the GII-4 VP1.

Further, the signal intensity ratio of GII-4 VP1, which was detected as a band of about 60k Da, to the sum of the signal intensity of all bands in each lane was measured by using a densitometer. As a result, while the ratio of the cell pellet cultivated for 3 days was 28%, the ratio of the culture supernatant cultivated for 6 days was 62%. Thus, the culture supernatant cultivated for 6 days contained less impurity than the cell pellet cultivated for 3 days.

### Example 2: measurement of cell viability and the like

The cells (expresSF+cells) were cultivated by using a bioreactor. PSFM medium was used as a medium, and the cultivation temperature was 27 °C. The culture solution was collected at just before the infection of the baculovirus expressing GII-4 VP1 and at 1-6 days after the infection. The viable cell number, the total cell number, the cell diameter, and the viability were measured by an automated cell viability analyzer (Vi-CELL XR, Beckman Coulter).

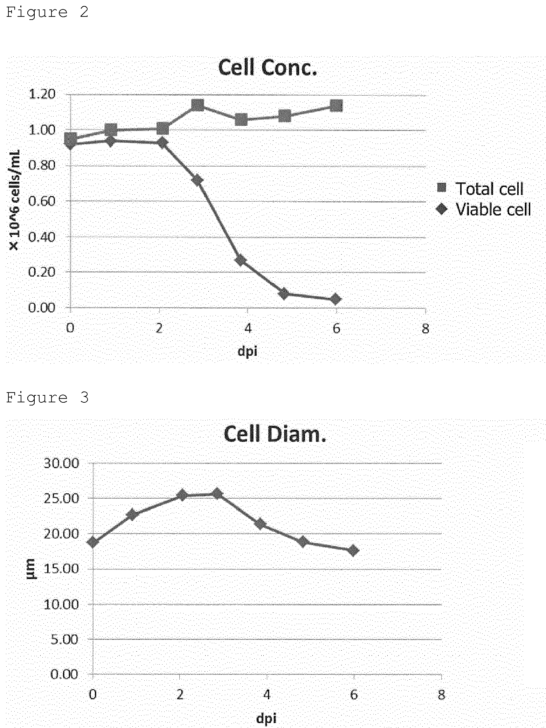

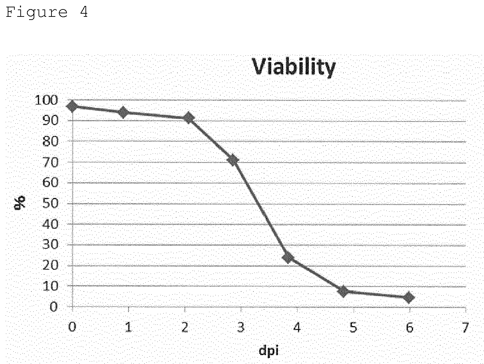

The temporal change of viable cell number and total cell number was shown in Figure 2. The temporal change of cell diameter was shown in Figure 3. The temporal change of viability was shown in Figure 4.

As shown in these figures, the viability of cells is rapidly reduced after 3 days from the start of the cultivation.

### INDUSTRIAL APPLICABILITY

Since the present invention is useful as production of vaccines against viruses such as norovirus, it can be utilized in an industrial field such as pharmaceutical industry.

## Claims

1. A production method for culture supernatant containing virus-like particles, comprising: transforming insect cells with baculovirus vector containing a viral nucleic acid sequence from norovirus, wherein the viral nucleic acid sequence encodes protein that can form a virus-like particle; cultivating the insect cells until the viability of the insect cells reaches 10% or less; and obtaining the culture supernatant.

2. The production method according to claim 1, wherein the cultivation period of the insect cells is 5 days or more.

3. The production method according to claim 1 or 2, wherein the viral nucleic acid sequence is a nucleic acid sequence encoding a norovirus structural protein VP1.

## Patentansprüche

1. Herstellungsverfahren für Kulturüberstand-enthaltende virusähnliche Partikel, umfassend: Transformieren von Insektenzellen mit einem Baculovirus-Vektor, der eine virale Nukleinsäuresequenz von Norovirus enthält, wobei die virale Nukleinsäuresequenz Protein codiert, das ein virusähnliches Partikel bilden kann; Kultivieren der Insektenzellen, bis die Viabilität der Insektenzellen 10 % oder weniger erreicht; und Erhalten des Kulturüberstands.

2. Herstellungsverfahren nach Anspruch 1, wobei die Kultivierungszeit der Insektenzellen 5 Tage oder mehr beträgt.

3. Herstellungsverfahren nach Anspruch 1 oder 2, wobei die virale Nukleinsäuresequenz eine Nukleinsäuresequenz ist, die ein Norovirus-Strukturprotein VP1 codiert.

## Revendications

1. Procédé d'obtention d'un surnageant de culture contenant des pseudo-particules virales, comprenant : la modification de cellules d'insecte par un vecteur de baculovirus contenant une séquence nucléotidique virale provenant d'un norovirus, où la séquence nucléotidique virale code la protéine qui peut former une pseudo-particule virale ; la culture des cellules d'insecte jusqu'à ce que la viabilité des cellules d'insecte soit inférieure ou égale à 10 % ; et l'obtention du surnageant de culture.

2. Procédé d'obtention selon la revendication 1, dans lequel la durée de la culture des cellules d'insecte est égale ou supérieure à 5 jours.

3. Procédé d'obtention selon la revendication 1 ou 2, dans lequel la séquence nucléotidique virale est une séquence nucléotidique codant une protéine structurale VP1 du norovirus.
